(19) Europäisches Patentamt
European Patent Office
Office européen des brevets

(11) EP 3 915 546 A1

(12) # EUROPÄISCHE PATENTANMELDUNG

(43) Veröffentlichungstag:
**01.12.2021 Patentblatt 2021/48**

(51) Int Cl.:
***A61K 9/14*** (2006.01) ***A61K 9/51*** (2006.01)

(21) Anmeldenummer: **20176307.5**

(22) Anmeldetag: **25.05.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **leon-nanodrugs GmbH**
**81679 München (DE)**

(72) Erfinder: **Horstkotte, Dr. Elke**
**81245 München (DE)**

(74) Vertreter: **Held, Stephan**
**Meissner Bolte Patentanwälte**
**Rechtsanwälte Partnerschaft mbB**
**Widenmayerstraße 47**
**80538 München (DE)**

(54) **VERWENDUNG VON SÄURE ALS LÖSUNGSMITTEL FÜR PRÄZIPITATIONEN**

(57) Die vorliegende Erfindung betrifft ein Verfahren zur Herstellung von Nanopartikeln, enthaltend mindestens einen pharmazeutisch aktiven Wirkstoff, umfassend die Schritte:
a) Bereitstellen einer Lösung des mindestens einen pharmazeutisch aktiven Wirkstoffs in einer Säure mit einem pks ≤ 5;
b) Bereitstellen eines Antilösungsmittels für den mindestens einen pharmazeutisch aktiven Wirkstoff; und
c) Ausfällen von Nanopartikeln, umfassend den mindestens einen pharmazeutisch aktiven Wirkstoff, durch Mischen der Lösung aus Schritt a) mit dem Antilösungsmittel aus Schritt b), um eine Suspension von Nanopartikeln zu erhalten, wobei die pharmazeutisch aktiven Wirkstoffe Tacrolimus und Nilotinib ausgeschlossen sind, die Nanopartikel erhältlich gemäß diesem Verfahren und eine pharmazeutische Zusammensetzung, umfassend diese Nanopartikel.

Figur 1

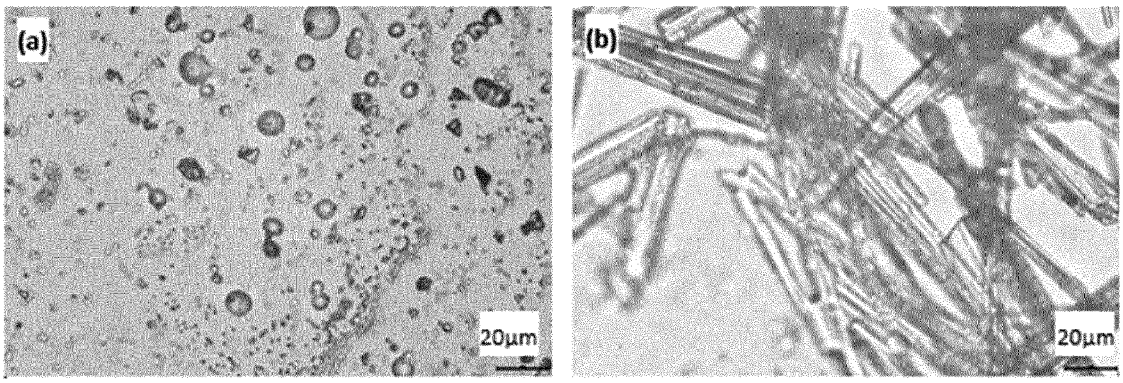


EP 3 915 546 A1

## Beschreibung

Die vorliegende Erfindung betrifft ein Verfahren zur Herstellung von Nanopartikeln, umfassend mindestens einen pharmazeutisch aktiven Wirkstoff, die Nanopartikel erhältlich gemäß diesem Verfahren und eine pharmazeutische Zusammensetzung enthaltend diese Nanopartikel.

Nanopartikel spielen bei der Entwicklung neuer Pharmazeutika eine bedeutende Rolle. Nanopartikel können aufgrund ihrer geringen Größe beispielsweise die Blut-Hirn-Schranke überwinden oder gezielt in Körper- oder Tumorzellen aufgenommen werden ("Drug-Targeting"). Unter bestimmten Umständen werden Nanopartikel vom Körper nicht als Fremdkörper erkannt und damit nicht vom Immunsystem eliminiert. Anwendungen von nanotechnologisch hergestellten Arzneimitteln sind in vielen Fachgebieten der Medizin möglich. Arzneiformen, die nanoskalige Wirkstoffe enthalten, können in vielen Bereichen der Medizin eingesetzt werden. So können damit Fortschritte in der Heilung und Diagnostik verschiedenster Erkrankungen erzielt werden: Es gibt bereits eine Reihe von Anwendungsgebieten, in denen Patente erteilt oder Medikamente auf dem Markt bzw. in der klinischen Erprobung sind: unter anderem bei malignen Tumoren, Infektionen, Stoffwechsel-Erkrankungen wie Diabetes, Autoimmun-Erkrankungen, Transplantat-Abstoßung und bei der Therapie von Entzündung und Schmerz (i. European Science Foundation, Nanomedicine - An ESF - European Medical Research Councils (EMRC) Forward Look report, Duncan, R. et al., 2004, European Science Foundation: Straßburg. p. 1-48.; ii. Couvreur, P. and C. Vauthier, Nanotechnology: intelligent design to treat complex disease, Pharm Res, 2006, 23(7): p. 1417-50; iii. Damge, C., C.P. Reis, and P. Maincent, Nanoparticle strategies for the oral delivery of insulin, Expert Opin Drug Deliv, 2008, 5(1): p. 45-68). Weiterhin können nanotechnologische Formulierungen in der Dermatologie eingesetzt werden (Schafer-Korting, M., W. Mehnert, and H.C. Korting, Lipid nanoparticles for improved topical application of drugs for skin diseases, Adv Drug Deliv Rev, 2007, 59(6): p. 427-43). Ein weiteres Anwendungsgebiet ist die Diagnostik in verschiedenen Bereichen der Medizin, insbesondere aber in der Onkologie. Nanotechnologische Arzneiformen können folgende Vorteile gegenüber herkömmlich hergestellten Arzneimitteln haben (Vauthier, C. and P. Couvreur, Developing nanoparticle drug carriers, Pharmaceutical technology europe, 2007(1): p. 35-42):

- Verbesserung der Löslichkeit und Bioverfügbarkeit des Wirkstoffes,
- Erhöhung der Aufnahme (Resorption) des Wirkstoffes, z.B. bei Arzneimitteln, die auf der Haut angewendet werden,
- Stabilisierung von neuartigen, aktiven Wirkstoffen (z. B. Peptide, Proteine),
- gezielter Transport von Wirkstoffen, z. B. zu Krebszellen ("Drug Targeting") oder in das ZNS (Schmerztherapie),
- kontrollierte Wirkstoffabgabe an den Körper über längere Zeit ("Controlled drug release"),
- Ermöglichung spezieller Diagnoseverfahren, z. B. in der Tumordiagnostik.

Nanopartikel lassen sich durch verschiedene Verfahren herstellen. Ein Verfahren besteht beispielsweise darin, Partikel durch gezielte Fällung aus übersättigten Lösungen zu generieren.

Zur Erzeugung von Nanopartikeln von schwer wasserlöslichen pharmazeutischen Wirkstoffen wird der betreffende Wirkstoff üblicherweise in einem organischen Lösungsmittel gelöst, z.B. Ethanol, Aceton, Tetrahydrofuran.

Die Herstellung der Nanopartikel erfolgt vorzugsweise durch Vermischung dieser organischen Wirkstoff-Lösung mit einem Antilösungsmittel, vorzugsweise Wasser, z.B. in einem MikroJetReaktor.

Für die pharmazeutische Weiterverarbeitung der Suspension zu einem Arzneimittel ("Downstream Processing") ist es wünschenswert, eine Wirkstoffkonzentration von beispielsweise mehr als 50 mg/ml zu erzeugen. Eine hohe Konzentration in der Suspension wird durch eine hohe Löslichkeit im Lösungsmittel und/oder durch Vermischung mit möglichst wenig Antilösungsmittel erreicht.

Die (Rest-)-Löslichkeit des Wirkstoffs in der erzeugten Suspension trägt wesentlich zu Partikelwachstum und damit Instabilität der Suspension bei. Daher ist eine vernachlässigbar kleine Löslichkeit des Wirkstoffs in der Suspension wünschenswert.

Die Aufgabe der vorliegenden Erfindung war es daher ein kostengünstiges und möglichst universelles Lösungsmittel für pharmazeutisch aktive Wirkstoffe, in dem sich der Wirkstoff in hoher Konzentration löst und dessen Lösevermögen bei der Mischung mit weitestgehend Wasser verloren geht, bereitzustellen und Nanopartikeln durch Fällung aus einem der Lösungsmittel, das obige Anforderungen erfüllt, herzustellen.

Ferner war es eine Aufgabe der vorliegenden Erfindung das Wachstum (Oswald Reifung) der durch Präzipitation erzeugten Nanopartikel in dem Gemisch, in dem eine kleine Löslichkeit besteht, auf vernachlässigbar kleine Werte zu reduzieren und damit eine deutliche Erhöhung der Stabilität der Suspension zu erreichen.

Überraschenderweise wurden Säuren mit einem pks $\leq 5$ als geeignete Lösungsmittel identifiziert. Insbesondere organische Säuren mit einem pks $\leq 5$, wie Eisessig und Ameisensäure, sind exzellente polare, protische Lösungsmittel für viele organische Substanzen. Eisessig und Ameisensäure sind mit Wasser und den gebräuchlichsten organischen Lösungsmitteln in jedem Verhältnis mischbar. Bei Mischung mit Wasser verlieren Eisessig und Ameisensäure ihre Löseeigenschaften.

Damit wird die oben genannte Aufgabe insbesondere durch einen Verfahren gemäß Anspruch 1 gelöst, also

durch ein Verfahren zur Herstellung von Nanopartikeln, enthaltend mindestens einen pharmazeutisch aktiven Wirkstoff, umfassend die Schritte:

a) Bereitstellen einer Lösung des mindestens einen pharmazeutisch aktiven Wirkstoffs in einer Säure mit einem pks ≤ 5;
b) Bereitstellen eines Antilösungsmittels für den mindestens einen pharmazeutisch aktiven Wirkstoff; und
c) Ausfällen von Nanopartikeln, umfassend den mindestens einen pharmazeutisch aktiven Wirkstoff, durch Mischen der Lösung aus Schritt a) mit dem Antilösungsmittel aus Schritt b), um eine Suspension von Nanopartikeln zu erhalten, wobei die pharmazeutisch aktiven Wirkstoffe Tacrolimus und Nilotinib ausgeschlossen sind.

Unter Tacrolimus (TAC, MW 822 g/mol) versteht man den immunsuppressiven Wirkstoff, der folgenden Formel,

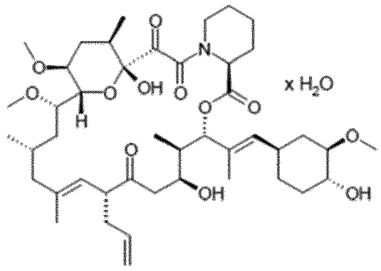

der zur Vorbeugung einer Transplantatabstossung und zur Behandlung von Abstossungsreaktionen eingesetzt wird. Tacrolimus ist ein komplexes Makrolid mit einem 23-gliedrigen Ring, das vom pilzähnlichen Bakterium Streptomyces tsukubaensis gebildet wird.

Nilotinib ($C_{28}H_{22}F_3N_7O$, MW 529,5 g/mol) ist ein spezifischer BCR-ABL-Tyrosinkinase-Inhibitor der folgenden Formel

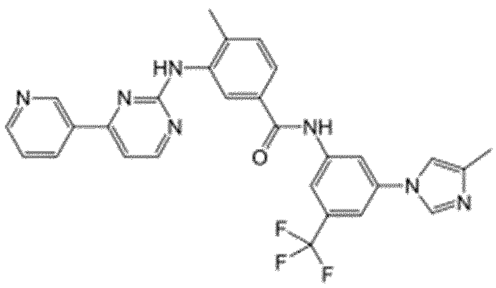

für die Behandlung der chronischen myeloischen Leukämie (CML).

In dem Lösungsmittel, das in Schritt a) eingesetzt wird und/oder dem Antilösungsmittel, das in Schritt b) eigensetzt wird, kann zusätzlich ein Oberflächenmodifikator, beispielsweise ein Tensid, enthalten sein.

Die Verben "umfassen" und "enthalten" und ihre Konjugationen umfassen das Verb "bestehen aus" und seine Konjugationen. Die Begriffe "ein" oder "eine" umfassen den Begriff "mindestens ein(e)". Ausführungsformen der Erfindung können in beliebiger Weise kombiniert werden, es sei denn, dies ist eindeutig nicht beabsichtigt.

Als Nanopartikel werden Partikel definiert, die mit einer Partikelgröße kleiner als 1000 nm gezielt hergestellt und verarbeitet werden.

Unter Säuren werden hier, wie dem Fachmann bekannt alle chemischen Verbindungen, die in der Lage sind, Protonen ($H^+$) an einen Reaktionspartner zu übertragen, verstanden.

Zwischen einer Säure HA und ihrer Base $A^-$ liegt in wässriger Lösung folgende Gleichgewichtsreaktion vor:

$$HA + H_2O \rightleftarrows H_3O^+ + A^-$$

Nach dem Massenwirkungsgesetz wird die Lage des Gleichgewichtes durch die Gleichgewichtskonstante K beschrieben:

$$\frac{c(H_3O^+) \cdot c(A^-)}{c(HA) \cdot c(H_2O)} = K$$

Da die Konzentration von Wasser ($c(H_2O)$) bei der Reaktion praktisch konstant bleibt, lässt sich $c(H_2O)$ in die Konstante K einbeziehen. Damit ergibt sich schließlich die Säurekonstante $K_S$ mit der Einheit mol/l:

$$K_S = \frac{c(H_3O^+) \cdot c(A^-)}{c(HA)}$$

Der pK-Wert ist definiert als der negativ dekadische Logarithmus der Gleichgewichtskonstanten einer chemischen Reaktion. Er wird unter anderem dazu benutzt, um die Acidität bzw. Basizität von Säuren bzw. Basen bei bestimmten Temperaturen auszudrücken. Bei Säuren ist der jeweilige Säureexponent (pKs oder pKa) der negativ dekadische Logarithmus der Säurekonstanten pKs = -lg Ks.

Der Begriff "Antilösungsmittel" oder auch Antisolvens oder Nicht-Lösungsmittel beschreibt gemäß der vorliegenden Erfindung jede flüssige Substanz, die in der Lage ist, den mindestens einen pharmazeutisch aktiven Wirkstoff in Form von Nanopartikeln auszufällen. Daher sollte ein "Antilösungsmittel" im Sinne der vorliegenden Erfindung nicht eng ausgelegt werden, z.B. als eine Substanz, in der der mindestens eine pharmazeutisch aktive Wirkstoff komplett unlöslich ist. Vorzugsweise sollte das Antilösungsmittel nicht mehr als 1 mg/ml dem jeweiligen pharmazeutisch aktiven Wirkstoff auflösen.

Das erfindungsgemäße Verfahren ist ferner vorzugsweise dadurch gekennzeichnet, dass das Mischen in Schritt c) das Kollidieren eines Stroms der Lösung a) mit einem Strom des Antilösungsmittels b) umfasst.

Für das Mischen in Schritt c), das das Kollidieren eines Stroms der Lösung a) mit einem Strom des Antilösungsmittels b) umfasst, wird vorzugsweise ein MicroJetReactor eingesetzt.

Ein MicroJetReactor (MJR) ist zum Beispiel aus der EP 1 165 224 B1, der EP 1 165 224 A2 oder der WO 2018/234217 A1 bekannt. Bei einem MicroJetReactor werden üblicherweise zwei Ströme vermischt bzw. kollidieren miteinander. Entsprechend kann die Anlage weitere Bauteile umfassen, wie sie beispielsweise in der EP 1 165 224 B1, der EP 1 165 224 A2 oder der WO 2018/234217 A1 beschrieben sind.

Die Herstellung von Nanopartikeln in einem MicroJetReactor ist in allen Parametern leicht beherrschbar und lässt sich auch in größeren Produktionsmaßstäben effizient und kostengünstig umsetzen. In einer MJR-Anlage fördern Pumpen ein Lösungsmittel mit einem darin enthaltenen Wirkstoff und ein Antilösungsmittel zu je einer Eintrittsdüse mit Durchmessern zwischen 50 und 1500 $\mu$m (oder kleiner als 50 $\mu$m, z.B. 1 $\mu$m). Dort baut sich ein hydrodynamischer Druck auf, der in Abhängigkeit von dem Durchmesser der Düse und der Pumprate 100 bar und mehr erreichen kann. Bei Durchtritt durch die Düse wird die Flüssigkeit im Inneren der Reaktorkammer auf Geschwindigkeiten von beispielsweise 1 bis 500 m/s beschleunigt. In der Reaktorkammer treffen die Flüssigkeiten in einem Kollisionspunkt aufeinander und durchmischen sich aufgrund der hohen kinetischen Energie intensiv. Der Wirkstoff fällt aufgrund der Verdünnung des Lösungsmittels und einer Übersättigung des Lösungsmittelgemisches als Partikel aus.

Das erfindungsgemäße Verfahren ist vorzugsweise dadurch gekennzeichnet, dass das Mischen in Schritt c) das Kollidieren eines Stroms der Lösung a) mit einem Strom des Antilösungsmittels b) umfasst, wobei die beiden Ströme bei einer Geschwindigkeit von 1 m/s bis 500 m/s, vorzugsweise bei 1 m/s bis 200 m/s, kollidieren. Hauptsächlich wird die Geschwindigkeit des ersten Stromes und des zweiten Stromes durch die jeweilige Pumprate sowie den Durchmesser der Eintrittsdüse des Reaktors bewirkt.

Das erfindungsgemäße Verfahren ist ferner vorzugsweise dadurch gekennzeichnet, dass die Säure mit einem pks $\leq$ 5 im Wesentlichen wasserfrei ist.

Unter im Wesentlichen wasserfrei wird dabei ein Wassergehalt von weniger als 2 Vol.-%, vorzugsweise von weniger als 1 Vol.-%, in der Säure, verstanden.

Als unter im Wesentlichen wasserfrei wird ebenfalls ein Wassergehalt von weniger als 2 Gew.-%, vorzugsweise von weniger als 1 Gew.-%, in der Säure, verstanden.

Der Einsatz einer im Wesentlichen wasserfreien Säure mit einem pks $\leq$ 5 hat den Vorteil, dass die Löslichkeit des mindestens einen pharmazeutischen Wirkstoffs gegenüber einer Säure, die mit Wasser verdünnt ist, erhöht ist.

Das erfindungsgemäße Verfahren ist ferner vorzugsweise dadurch gekennzeichnet, dass die Säure mit einem pks $\leq$ 5 eine organische Säure, vorzugsweise im Wesentlichen wasserfrei, mit einem pks $\leq$ 5 ist.

Unter organische Säure wird hierbei eine Verbindung der allgemeinen Formel

R-XH

verstanden, wobei X ein Heteroatom, wie O, N, P oder S umfasst und R einen organischen Rest, vorzugsweise einen Kohlenwasserstoffrest, der optional subsituiert ist, umfasst und wobei die organische Säure einen pks ≤ 5 aufweist.

Bevorzugte Vertreter organischer Säuren sind Carbonsäuren, subsituierte Carbonsäuren, aromatische Carbonäuren, Sulfonsäuren und/oder Phosphor- bzw. Phosphonsäuren.

In einer besonders bevorzugten Ausführungsform ist das erfindungsgemäße Verfahren dadurch gekennzeichnet, dass die Säure mit einem pks ≤ 5 Eisessig und/oder Ameisensäure, vorzugsweise im Wesentlichen wasserfrei, ist.

Unter Eisessig wird wie allgemein üblich Ethansäure bzw. reine Essigsäure verstanden. Unter Ameisensäure wird wie allgemein üblich Methansäure verstanden.

Eisessig und Ameisensäure haben den Vorteil, dass eine Vielzahl von pharmazeutisch aktiven Wirkstoffen darin sehr gut löslich ist. Ferner sind Eisessig und Ameisensäure kostengünstig und in beliebiger Menge verfügbar.

In einer weiteren bevorzugten Ausführungsform ist das erfindungsgemäße Verfahren dadurch gekennzeichnet, dass das Antilösungsmittel Wasser umfasst oder Wasser ist.

Vorzugsweise ist das Antilösungsmittel Wasser.

Es sind auch Ausführungsformen möglich, wobei das Antilösungsmittel eine Mischung aus Wasser und organischen Lösungsmitteln, wie beispielsweise Alkoholen oder Ketonen, ist oder umfasst. Vorzugsweise umfasst das organische Lösungsmittel in solchen Ausführungsformen Methanol, Ethanol, Acetonitril, Dimethylsulfoxid, Aceton, Propan-1-ol, Propan-2-ol und/oder tert.-Butylalkohol.

Das erfindungsgemäße Verfahren ist ferner vorzugsweise dadurch gekennzeichnet, dass die Konzentration des mindestens einen pharmazeutisch aktiven Wirkstoffs in der Suspension mindestens 50 mg/ml, vorzugsweise 80 mg/ml bis 300 mg/ml, besonders bevorzugt 50 mg/ml bis 100 mg/ml beträgt.

Solche Wirkstoffkonzentrationen sind für die pharmazeutische Weiterverarbeitung der Suspension zu einem Arzneimittel ("Downstream Processing") besonders vorteilhaft.

Der mindestens eine pharmazeutisch aktive Wirkstoff ist prinzipiell nicht beschränkt. Vorzugsweise umfasst der mindestens eine pharmazeutisch aktive Wirkstoff Itraconazol, Enzalutamide, Aprepitant sowie pharmazeutisch akzeptable Salze davon.

Die pharmazeutisch aktiven Wirkstoffe Tacrolimus und Nilotinib sind dabei gemäß der vorliegenden Erfindung ausgeschlossen.

Itraconazol (ITZ, $C_{35}H_{38}C_{12}N_8O_4$, MW 705.6 g/mol) ist ein Wirkstoff aus der Gruppe der Triazol-Antimykotika zur Behandlung von Pilzinfektionen mit verschiedenen Erregern mit der folgenden Formel.

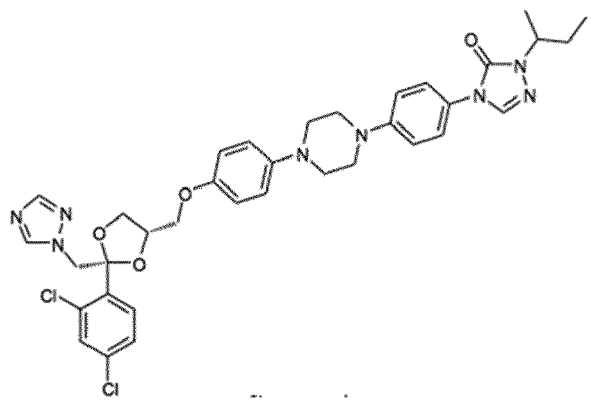

Itraconazol ist unter anderem gegen Hefen, Dermatophyten und Schimmelpilze wirksam. ITZ ist in Wasser praktisch unlöslich. Die Löslichkeit von ITZ ist pH-Wert abhängig und damit in Säuren gut löslich.

Enzalutamid (ENZ, $C_{21}H_{16}F_4N_4O_2S$, MW 464,44 g/mol) ist ein pharmazeutischer Wirkstoff zur Behandlung von Patienten mit metastasiertem Prostatakarzinom mit der folgenden Formel.

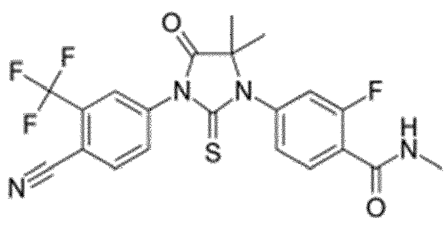

Enzalutamid ist ein Molekül ohne ionisierbare Gruppen im biologisch relevantem pH-Wert Bereich. Daher wird die Löslichkeit von Enzalutamid durch den pH-Wert im physiologischen Bereich nicht beeinflusst. Die Substanz ist praktisch nicht in Wasser löslich und unabhängig vom pH-Wert. In reinen Säuren, insbesondere in Ameisensäure und Eisessig ist ENZ gut löslich. Bei Zugabe von Wasser sinkt die Löslichkeit stark ab.

Aprepitant (APR, $C_{23}H_{21}F_7N_4O_3$, MW 534,4 g/mol) ist ein Wirkstoff aus der Gruppe der Gruppe der Neurokinin-1-Rezeptor-Antagonisten der folgenden Formel,

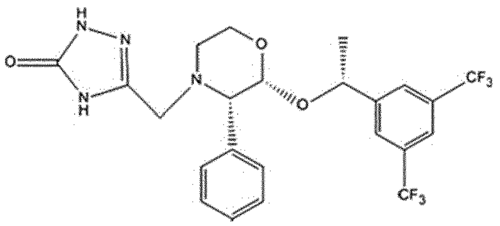

der als Antiemetikum zur Verhütung von akuter und verzögerter Übelkeit und Erbrechen bei hoch emetogener Chemotherapie eingesetzt wird.

Aprepitant wird unter dem Handelsnamen "EMEND" als Kapsel für die orale Einnahme mit 125 mg, 80 mg und 40 mg Wirkstoffgehalt vertrieben. EMEND enthält Aprepitant in Form von Nanopartikeln, die durch ein Nassmahlverfahren hergestellt werden. Nach WO 2003 049718 ist es wichtig, auf der Oberfläche der Aprepitant Nanopartikel Oberflächenmodifikatoren physikalisch zu adsorbieren, um so Wachstum und Aggregation der Partikel zu verhindern. Bei der Disintegration der Kapsel (re-)dispergieren die Nanopartikel aus dem Feststoff.

APR ist in Wasser zwischen pH-Werten von 2 bis 12 praktisch unlöslich. In Säuren, insbesondere in Ameisensäure oder Eisessig ist APR gut löslich. Durch Fällung aus einer Säure lassen sich Nanopartikel von Aprepitant herstellen, die überraschenderweise auch ohne Oberflächenmodifikator dispergieren und vergleichbare Dispersionswerte zu EMEND ergeben. Vorzugsweise wird daher bei der Herstellung von Nanopartikeln, umfassen Aprepitant kein Oberflächenmodifikator eingesetzt.

Oberflächenmodifikatoren sind vorzugsweise Substanzen, die dem Lösungsmittel und/oder dem Antilösungsmittel zugegeben werden und die nach Bildung der Nanopartikel auf der Oberfläche der Nanopartikel vorliegen und die Nanopartikel dadurch stabilisieren.

In einer besonders bevorzugten Ausführungsform umfasst oder ist der mindestens eine pharmazeutisch aktive Wirkstoff Aprepitant und das zugehörige Lösungsmittel ist Ameisensäure und/oder Eisessig, besonders bevorzugt Eisessig, wobei in der Ameisensäure und/oder dem Eisessig kein Oberflächenmodifikator zugegeben wird und wobei auch dem Antilösungmittel kein Oberflächenmodifikator zugegeben wird.

Die so erhaltenen Nanopartikel von AR enthalten somit vorzugsweise keinen Oberflächenmodifikator, insbesondere nicht auf der Oberfläche der Nanopartikel.

Bekannte Oberflächenmodifikatoren umfassen beispielsweise synthetische oder natürliche Polymere, sowie Tenside.

Bekannte Oberflächenmodifikatoren umfassen insbesondere Polymere, wie Polyvinylpyrrolidone (PVP), Vinylpyrrolidon-/Vinylacetat-Copolymere, Polyethylenglycole und/oder Cellulosederivate wie Hydroxypropylmethylcellulose (HPMC), Hydroxypropylmethylcellulosephthalat (HPMCP), Hydroxypropylmethylcelluloseacetatsuccinat (HPMCAS), Hydroxypropylcellulose (HPC), Ethylcellulose (EC) und/oder Carboxymethylcellulose (CMC).

Weitere bekannte Oberflächenmodifikatoren umfassen insbesondere einen Sorbitanfettsäureester, wie Sorbitanmonostearat (Span 60), Sorbitantristearat (Span 65) oder Sorbitanmonolaurat (Span 20), einen ethoxylierten Sorbitanfettsäureester, wie Polysorbat 20 (Tween 20), Polysorbat 80 (Tween 80), Propylenglycolmonolaurat (type II) (Lauroglycol™ 90, Gattefosse), Propylenglycolmonolaurat (type I) (Lauroglycol™ FCC, Gattefosse), (Polyglyceryl-3-dioleat (Plurol® Oleique CC 497, Gattefosse), Oleoyl-macrogol-6 Glyceride (Labrafil® M 1944 CS, Gattefosse), Linoleoyl-macrogol-6 Glyceride (Labrafil® M 2125 CS, Gattefosse), Lauroyl-macrogol-6 Glyceride (Labrafil® M 2130 CS, Gattefosse), Lauroyl-macrogol-32 Glyceride (Gelucire® 44/14, Gattefosse), Macrogolstearat (Gelucire® 48/16, Gattefosse), Stearoyl-macrogol-32 Glyceride (Gelucire 50/13, Gattefosse), Caprylocaproyl-macrogol-8 Glyceride (Labrasol® ALF, Gattefosse), Vitamin E-polyethylenglycolsuccinat (Kolliphor TPGS, BASF), Poloxamer 407 (Kolliphor P 407, BASF), Poloxamer 188 (Kolliphor P 188, BASF), ein anionisches Tensid, wie Natriumdocusat, Natriumlaurylsulfat, Natriumoleat, Natriumdeoxycholat, Natriumstearat oder ein kationisches Tensid, wie Hexadecyltrimethylammoniumbromid (CTAB).

Geeignete Oberflächenmodifikatoren aus der Klasse der Polymere, wie zum Beispiel (Meth)acrylsäure/Methyl(meth)acrylat-Copolymere, (Meth)acrylsäure/Ethyl(meth)acrylat-Copolymere, Glycerolpolyethylenglycolricinoleate, Polyethylenglycole, Polyethylenglycol/Polyvinylacetat/Polyvinylcaprolactam basierte Graft-Copolymere, Polyvinylpyrrolidone und Poly(1-vinylpyrrolidon-co-vinylacetat)e, sind unter den folgenden Handelsnamen bekannt, Eudragit® E PO,

Eudragit® FS 30 D, Eudragit® L 12,5, Eudragit® FS 30, Eudragit® RS PO, Eudragit® RL PO, Kolliphor HS 15, Kolliphor RH 40, Kollisolv P 124, Soluplus, Povidon K17, Povidon K25, Povidon K30, Povidon K60, Povidon K90 und Copovidone.

In einer bevorzugten Ausführungsform umfasst oder ist der mindestens eine pharmazeutisch aktive Wirkstoff Itraconazol und zugehörige das Lösungsmittel ist Ameisensäure und/oder Eisessig, besonders bevorzugt Eisessig.

In einer bevorzugten Ausführungsform umfasst oder ist der mindestens eine pharmazeutisch aktive Wirkstoff Enzalutamid und zugehörige das Lösungsmittel ist Ameisensäure und/oder Eisessig, besonders bevorzugt Eisessig.

In einer bevorzugten Ausführungsform umfasst oder ist der mindestens eine pharmazeutisch aktive Wirkstoff Tacrolimus und zugehörige das Lösungsmittel ist Ameisensäure und/oder Eisessig, besonders bevorzugt Eisessig.

In einer bevorzugten Ausführungsform umfasst oder ist der mindestens eine pharmazeutisch aktive Wirkstoff Aprepitant und zugehörige das Lösungsmittel ist Ameisensäure und/oder Eisessig, besonders bevorzugt Eisessig.

Das erfindungsgemäße verfahren ist ferner vorzugsweise dadurch gekennzeichnet, dass das Volumenverhältnis der Lösung, umfassend den mindestens einen pharmazeutisch aktiven Wirkstoff aus Schritt a) zu dem Antilösungsmittel aus Schritt b) von 1:1 bis 1:10, vorzugsweise von 1:1 bis 1:5, beträgt.

Das erfindungsgemäße Verfahren ist ferner vorzugsweise dadurch gekennzeichnet, dass das Antilösungsmittel aus Schritt b) mindestens ein ionisches und/oder nicht-ionische Tensid, vorzugsweise einen Sorbitanfettsäureester, wie Sorbitanmonostearat (Span 60), Sorbitantristearat (Span 65) oder Sorbitanmonolaurat (Span 20), einen ethoxylierten Sorbitanfettsäureester, wie Polysorbat 20 (Tween 20), Polysorbat 80 (Tween 80), Propylenglycolmonolaurat (type II) (Lauroglycol™ 90, Gattefosse), Propylenglycolmonolaurat (type I) (Lauroglycol™ FCC, Gattefosse), (Polyglyceryl-3-dioleat (Plurol® Oleique CC 497, Gattefosse), Oleoyl-macrogol-6 Glyceride (Labrafil® M 1944 CS, Gattefosse), Linoleoyl-macrogol-6 Glyceride (Labrafil® M 2125 CS, Gattefosse), Lauroyl-macrogol-6 Glyceride (Labrafil® M 2130 CS, Gattefosse), Lauroyl-macrogol-32 Glyceride (Gelucire® 44/14, Gattefosse), Macrogolstearat (Gelucire® 48/16, Gattefosse), Stearoyl-macrogol-32 Glyceride (Gelucire 50/13, Gattefosse), Caprylocaproyl-macrogol-8 Glyceride (Labrasol® ALF, Gattefosse), Vitamin E-polyethylenglycolsuccinat (Kolliphor TPGS, BASF), Poloxamer 407 (Kolliphor P 407, BASF), Poloxamer 188 (Kolliphor P 188, BASF), ein anionisches Tensid, wie Natriumdocusat, Natriumlaurylsulfat, Natriumoleat, Natriumdeoxycholat, Natriumstearat oder ein kationisches Tensid, wie Hexadecyltrimethylammoniumbromid (CTAB), umfasst.

Das mindestens ein ionisches und/oder nicht-ionische Tensid, vorzugsweise Natriumdodecylsulfat und/oder Natriumdocusat, liegt in dem Antilösungsmittel vorzugsweise in einer Menge von 0,1 bis 20 fachen Verhältnis zum Wirkstoff (nach Mischung), vorzugsweise von 0,05 bis 10 fachen Verhältnis, vor.

Das mindestens eine ionische und/oder nicht-ionische Tensid liegt in den ausgefällten Nanopartikeln vorzugsweise nicht auf der Oberfläche der Nanopartikel vor.

Das mindestens eine ionische und/oder nicht-ionische Detergenz liegt in den ausgefällten Nanopartikeln vorzugsweise im Kern der Nanopartikel, vorzugsweise ausschließlich im Kern der Nanopartikel, vor oder ist aufgrund seiner amphiphilen Eigenschaften auch oder ausschließlich auf der Oberfläche der Nanopartikel adsorbiert

In einer bevorzugten Ausführungsform ist das erfindungsgemäße Verfahren dadurch gekennzeichnet, dass die Nanoartikel eine durchschnittliche Partikelgröße, gemessen mittels dynamischer Lichtstreuung, von 10 bis 500 nm, vorzugsweise von 10 bis 200 nm aufweisen.

In einer weiteren bevorzugten Ausführungsform ist das erfindungsgemäße Verfahren dadurch gekennzeichnet, dass die Nanoartikel einen Polydispersitätsindex von $\leq 0,4$, bevorzugt von $\leq 0,2$, aufweisen.

Die Partikelgröße ("z-average") und die Größenverteilung bzw. der Polydispersitätsindex ("PDI") der ausgefällten Partikel wird vorzugsweise bei 25°C durch dynamische Lichtstreuung (DLS) bestimmt, vorzugsweise mit einem Zetasizer Nanoseries von Malvern Panalytical Ltd., und nach ISO 13321:1996 und ISO 22412:2017 berechnet. Die Partikelgröße wird als "z-average" angegeben, die durch Cumulanten-Analyse der Autokorrelationsfunktion erhalten wird. Der Polydispersity Index ("PDI") gilt als Maß für die Breite der Verteilung und ist eine dimensionslose Größe zwischen 0 und 1. Je kleine diese Zahl, desto schmalbandiger ist die Verteilung. Werte unter 0,4 zeigen im Allgemeinen eine gewissermaßen homogene Partikelgrößenverteilung, während Werte größer 0,7 anzeigen, dass die Probe eine sehr inhomogene Größenverteilung aufweist. Die Software ist vorzugsweise Zetasizer V7.13. Als Messparameter werden vorzugsweise eingestellt:

i) Probe

Messprinzip: DLS Partikelgrößenverteilung
Probenmaterial: Polystyrollatexstandard
Brechungsindex des Probenmateriales: 1,590 (Polystyrollatexstandard bei 25 °C)
Absorption des Probenmateriales: 0,010
Dispersionsmedium: Wasser
Viskosität des Dispersionsmediums: 0,8872 mPa*s (Viskosität von Wasser bei 25 °C)
Temperatur: 25 °C

Äquilibrierungszeit: 60 s
Messzelle (Typ): Einwegküvette
Länge des optischen Pfades der Messzelle: 10 mm

ii) Messung

Position der Messzelle: Automatisch
Messwinkel: 173 °, Rückstreuung (Backscattering)
Messdauer: Manuell, Anzahl der Läufe: 15, Laufdauer 10 s
Anzahl der Messzyklen: drei, ohne Verzögerung dazwischen
Messmodus: Intensitätsmodus
Verzögerungszeit für große Partikel: Nein
Attenuation: Automatische Auswahl

Bevorzugt wird das Gerät vor der Bestimmung mit einer Dispersion eines Partikelgrößenstandards (etwa 100 nm) des Geräteherstellers validiert. Vorzugsweise werden die Partikelgrößen direkt (innerhalb von 30 Minuten) nach dem Ausfällen oder immer nach Ablauf einer festgelegten Zeit (beispielsweise 2 Minuten) bestimmt.

Die vorliegende Erfindung betrifft ferner Nanopartikel, erhältlich durch das vorstehend beschriebene Verfahren. Die erhaltenen Nanopartikel zeichnen sich insbesondere durch einen niedrigen PDI aus.

Die vorliegende Erfindung betrifft ferner eine pharmazeutische Zusammensetzung, umfassend die Nanopartikel erhältlich durch das vorstehend beschriebene Verfahren.

Die vorliegende Erfindung betrifft ferner die Verwendung einer pharmazeutischen Zusammensetzung, umfassend die Nanopartikel erhältlich durch das vorstehend beschriebene Verfahren als Arzneimittel.

Die vorliegende Erfindung betrifft ferner die Verwendung von Säure mit einem pks $\leq$ 5, vorzugsweise von einer organischen Säure mit einem pks $\leq$ 5, vorzugsweise von Ameisensäure und/oder Eisessig als Lösungsmittel in dem vorstehend beschriebenen Verfahren.

Beschreibung der Figuren:

Figur 1: Mikroskopische Aufnahme von ENZ, entstanden durch Mischen von 1 Teil einer Lösung von 100 mg/ml ENZ in Aceton mit 1 Teil Wasser. Maßstab: Der Balken im Bild entspricht 20 μm. (a) Aufnahme unmittelbar nach Fällung. (b) Aufnahme ca. 12 Stunden nach der Fällung.

Figur 2: Mikroskopische Aufnahme von ENZ-Partikeln. Entstanden durch Mischen von 1 Teil einer Lösung von 100 mg/ml ENZ in Eisessig mit 10 Teilen Wasser. Maßstab: Der Balken im Bild entspricht 20 μm. (a) Aufnahme ca. 1 Stunde nach Fällung. (b) Aufnahme ca. 18 Stunden nach der Fällung.

Die Erfindung wird nachfolgend anhand nicht beschränkender Beispiele näher erläutert.

Beispiele:

Zur Bestimmung der thermodynamischen Sättigungslöslichkeit einer Substanz in einem bestimmten Lösungsmittel wird zunächst zur Orientierung eine Literaturrecherche durchgeführt, um einen ersten groben Richtwert zu erhalten. Danach wurden 500 mg (und gegebenenfalls mehr) des Wirkstoffpulvers eingewogen und in 5 ml des Lösungsmittels unter leichtem Rühren suspendiert. Falls das Pulver vollständig in Lösung ging, wurde zusätzlich 100 mg (oder iterative nochmalige Zugabe der initial eingewogenen Menge) Feststoff zugesetzt. Dies wurde wiederholt, bis sich ein Bodensatz bildete.

Die feststoff-enthaltende Mischung wurde verschlossen und 24 Stunden bei Raumtemperatur gerührt. Nach 24 Stunden wurde der Überstand von Bodensatz getrennt, zentrifugiert (Filtration ist nahezu unmöglich) und zur Quantifizierung mit der mobilen Phase der HPLC-Methode verdünnt.

Ein weiterer Teil des zentrifugierten Überstandes wurde unter Rühren mit demineralisiertem Wasser im Volumenverhältnis von 1:1 gemischt. Dazu wurde Wasser vorgelegt und die Wirkstofflösung unter Rühren oder Schütteln zu pipettiert. Dabei fiel Wirkstoff aus. Der Überstand wurde nach 3 Stunden von Bodensatz getrennt, zentrifugiert und zur Quantifizierung mittels HPLC verdünnt. Der Verdünnungsschritt wird grob abgeschätzt, Zielkonzentration sind z. B. 100 μg/mL je nach Wirkstoff, um im Linearitätsbereich des Detektors zu bleiben. Liegt die Konzentration zu hoch oder

zu niedrig wird der Verdünnungsschritt angepasst. Die Verdünnung erfolgte mit einem zu mobilen Phase kompatiblen Lösungsmittelgemisch (z. B. Methanol vorlegen, Probe zugeben, Wasser zugeben). Falls die Substanz in diesem Lösungsmittelgemisch in dieser Verdünnung nicht löslich war, wurde die Verdünnung mit reinem organischem Lösungsmittel wiederholt.

Dieses Vorgehen wurde für die Mischungsverhältnisse von 1:2, 1:3 und 1:10 wiederholt.

Beispiel 1:

Die thermodynamische Sättigungslöslichkeit von ITZ in gängigen organischen Lösungsmitteln und deren Mischungen mit Wasser wurde nach dem vorstehend beschriebene Verfahren bestimmt. Die Ergebnisse sind in Tabelle 1 angegeben.

Tabelle 1: Sättigungslöslichkeit von ITZ in Ameisensäure, Eisessig, Tetrahydrofuran (THF), Aceton, Ethanol und in deren Mischungen mit Wasser in mg/ml und in Klammern relativ zu der Sättigungslöslichkeit im Lösungsmittel

| **Nr.** | **Lösungsmittel -Gehalt der Mischung** | **Sättiqunqslöslichkeit von ITZ** | | | | |
|---|---|---|---|---|---|---|
| | | **Ameisensäure** | **Eisessig** | **THF** | **Aceton** | **Ethanol** |
| | % | mg/ml (%) | mg/ml (%) | mg/ml (%) | mg/ml (%) | mg/ml (%) |
| 1 | 100 | 435 | 157,8 (100) | 4,255 (100) | 2,500 (100) | 0,289 (100) |
| 2 | 50 | n.b.(*) | 1,455 (0,92) | 0,677 (15,91) | 0,038 (1,53) | 0,102 (34,36) |
| 3 | 33,3 | n.b.(*) | 0,210 (0,13) | 0,013 (0,29) | 0,008 (0,34) | 0,002 (0,69) |
| 4 | 25 | n.b.(*) | 0,009 (0,01) | 0,002 (0,05) | 0,002 (0,09) | 0,006 (2,08) |
| 5 | 9,09 | 0,01 (0,002) | 0,028 (0,02) | 0,001 (0,02) | 0,0001 (0,004) | 0,009 (2,95) |
| (*) n.b. = nicht bekannt | | | | | | |

In den Lösungsmitteln Ameisensäure und Eisessig (Nr. 1 in Tabelle 1) löst sich ITZ in Konzentrationen >150 mg/ml, während sich ITZ in den gängigen organischen Lösungsmitteln Tetrahydrofuran, Aceton und Ethanol in sehr geringen Mengen löst (<5 mg/ml).

Bei Vermischung einer in einem organischen Lösungsmittel gesättigten Lösung von ITZ mit Wasser zur Ausfällung von Feststoff-Partikeln ergeben sich in der Suspension die in untenstehender Tabelle 2 ausgewiesenen Gesamtkonzentrationen.

Für die pharmazeutische Weiterverarbeitung der Suspension zu einem Arzneimittel ist es wünschenswert, eine Wirkstoffkonzentration von mehr als 50 mg/ml zu erzeugen. Dies ist nur für die beiden Lösungsmittel Ameisensäure und Eisessig möglich.

Tabelle 2: Erzeugung von ITZ Partikeln durch Fällung. Gesamtkonzentration an ITZ in der Suspension (mg/ml) und in Klammern der Anteil (%) des in der Mutterlauge gelösten ITZ

| **Nr.** | **LösungsmittelGehalt der Mischung** | **ITZ bei Fällung aus verschiedenen Lösungsmitteln** Gesamtkonzentration ITZ in mg/ml (davon in Lösung % in der Gesamtmenge in Suspension) | | | | |
|---|---|---|---|---|---|---|
| | % | **Ameisensäure** | **Eisessig** | **THF** | **Aceton** | **Ethanol** |
| 1 | 100 | 435,0 (100) | 157,8(100) | 4,25 (100) | 2,50 (100) | 0,29 (100) |
| 2 | 50 | 217,5 (n.b.) | 78,9 (1,84) | 2,13 (31,8) | 1,25 (3,07) | 0,14 (70,7) |
| 3 | 33,3 | 145,0 (n.b.) | 52,6 (0,40) | 1,42 (0,88) | 0,83 (1,02) | 0,10 (2,08) |
| 4 | 25 | 108,8 (n.b.) | 39,5 (0,02) | 1,06 (0,19) | 0,63 (0,35) | 0,07 (8,32) |

(fortgesetzt)

| Nr. | LösungsmittelGehalt der Mischung | ITZ bei Fällung aus verschiedenen Lösungsmitteln Gesamtkonzentration ITZ in mg/ml (davon in Lösung % in der Gesamtmenge in Suspension) | | | | |
|---|---|---|---|---|---|---|
| | % | **Ameisensäure** | **Eisessig** | **THF** | **Aceton** | **Ethanol** |
| 5 | 9,09 | 39,5 (0,03) | 14,3 (0,20) | 0,39 (0,19) | 0,23 0,04) | 0,03 (32,4) |
| (*) n.b. = nicht bekannt | | | | | | |

Die Sättigungslöslichkeit des Wirkstoffs in der Mutterlauge der Suspension trägt wesentlich zum Partikelwachstum und damit Instabilität der Suspension bei. Daher ist eine vernachlässigbar kleine Löslichkeit des Wirkstoffs in der Suspension wünschenswert.

Es wird eine gesättigte Lösung von 157,8 mg/ml ITZ in Eisessig mit zwei Teilen Wasser gemischt. Dabei fällt ein Großteil des ITZ aus, da dessen Sättigungskonzentration (0,210 mg/ml, Tabelle 1, Nr. 3, Eisessig) überschritten wird. Die resultierende Mischung enthält 33,3% Essig und eine Gesamtmenge von 52,6 mg/ml ITZ (Tabelle 2, Nr. 3, Eisessig). Davon werden 0,210 mg/ml in der Mutterlauge gelöst vorliegen; dies entspricht 0,40 % der Gesamtmenge von 52,6 mg/ml (Tabelle 2, Nr. 3, Eisessig).

Diese gelöste Menge an ITZ (0,40 % der Gesamtmenge) ist sehr klein und wird ein unerwünschtes Wachstum der Partikel hindern.

Beide Anforderungen von (1) einer Konzentration >50 mg/ml und (2) einer gelösten Menge an ITZ <1% sind nur für die Lösungsmittel Ameisensäure und Eisessig erfüllt.

Die Lösungsmittel THF, Aceton und Ethanol sind in diesem Beispiel ungeeignet.

Beispiel 2:

Die thermodynamische Sättigungslöslichkeit von ENZ in verschiedene Lösungsmitteln und deren Mischungen mit Wasser wurde nach dem vorstehend beschriebene Verfahren bestimmt. Die Ergebnisse sind in Tabelle 3 angegeben.

Tabelle 3: Sättigungslöslichkeit von ENZ in Eisessig, Tetrahydrofuran, Aceton und in deren Mischungen mit Wasser

| Nr. | LösungsmittelGehalt der Mischung | Sättigungslöslichkeit von ENZ | | |
|---|---|---|---|---|
| | | **Eisessig** | **THF** | **Aceton** |
| | % | mg/ml | mg/ml | mg/ml |
| 1 | 100 | 155,4 | 364 | 358 |
| 2 | 50 | 2,044 | 16,9 | 7,87 |
| 3 | 33,3 | 0,294 | 2,68 | 0,537 |
| 4 | 25 | 0,112 | 0,365 | 0,233 |
| 5 | 9,09 | 0,086 | n.b. (*) | n.b. (*) |
| (*) n.b. = nicht bekannt | | | | |

In dem Lösungsmittel Eisessig (Nr. 1 in Tabelle 3) löst sich ENZ einer Konzentrationen >150 mg/ml.

Bei Vermischung einer organischen Lösung von 150 mg/ml ENZ mit Wasser zur Ausfällung von Feststoff-Partikeln ergeben sich in der Suspension die in untenstehender Tabelle 4 ausgewiesenen Konzentrationen. Für die pharmazeutische Weiterverarbeitung der Suspension zu einem Arzneimittel ist es wünschenswert, eine Wirkstoffkonzentration von mehr als 50 mg/ml zu erzeugen.

Tabelle 4: Erzeugung von ENZ Partikeln durch Fällung. Gesamtkonzentration an ENZ in der Suspension (mg/ml) und der Anteil (%) des in der Mutterlauge gelösten ENZ

| Nr . | Lösungsmittel -Gehalt der Suspension | ENZ in Suspension | ENZ in Lösung bei Fällung aus (% der Gesamtmenge in Suspension) | | |
|---|---|---|---|---|---|
| | % | mg/ml | **Eisessig** | **THF** | **Aceton** |
| 1 | 100 | 100 | 100 | 100 | 100 |

(fortgesetzt)

| Nr . | Lösungsmittel -Gehalt der Suspension | ENZ in Suspension | ENZ in Lösung bei Fällung aus (% der Gesamtmenge in Suspension) | | |
|---|---|---|---|---|---|
| | % | mg/ml | Eisessig | THF | Aceton |
| 2 | 50 | 50 | 4,09 | 33,8 | 15,7 |
| 3 | 33,3 | 33,3 | 0,88 | 8,04 | 1,61 |
| 4 | 25 | 25 | 0,45 | 1,46 | 0,93 |
| 5 | 9,09 | 9,1 | 0,95 | n.b. (*) | n.b. (*) |
| (*) n.b. = nicht bekannt | | | | | |

Fällung von ENZ aus Aceton im Verhältnis 1:1

100 mg Enzalutamide werden unter Rühren in 1 ml Aceton bei Raumtemperatur gelöst (100 mg/ml). Diese Lösung wird in einem MikroJetReaktor mit Wasser gemischt in einem Mischungsverhältnis von 1 Teil Enzalutamide in Aceton zu 1,01 Teilen Wasser. Die erhaltene Suspension wird mikroskopiert (Figur. 1). Die Fällung liefert Mikropartikel, die innerhalb von 12 Stunden zu großen Nadeln wachsen.

Wird eine Lösung von 100 mg/ml Enzalutamide in Aceton mit gleichem Teil an Wasser gemischt, so fällt ein Großteil des Enzalutamides aus, da dessen Sättigungskonzentration überschritten wird. Die resultierende Mischung enthält 50% Aceton und eine Gesamtmenge von 50 mg/ml Enzalutamide. Davon werden 7,87 mg/ml in der Mutterlauge gelöst vorliegen (Tabelle 3, Nr. 2, Aceton); dies entspricht 15,7 % der Gesamtmenge von 50 mg/ml (Tabelle 4, Nr. 2, Aceton). Diese gelöste Menge an Enzalutamide (15,7 % der Gesamtmenge) ist zu hoch und führt zu einem unerwünschten und schnellen Wachstum der Partikel.

Fällung von ENZ aus Eisessig im Verhältnis 1:10

100 mg Enzalutamide werden unter Rühren in 1 ml Eisessig bei Raumtemperatur gelöst (100 mg/ml). Diese Lösung wird in einem MikroJetReaktor mit Wasser gemischt in einem Mischungsverhältnis von 1 Teil Enzalutamide in Eisessig zu 10 Teilen Wasser. Die erhaltene Suspension wird mikroskopiert (Figur 3). Die Fällung liefert im Vergleich zu Aceton deutlich kleinere Partikel, die in Laufe von 18 Stunden zu Nadeln wachsen.

Wird eine Lösung von 100 mg/ml Enzalutamide in Eisessig mit gleichem Teil an Wasser gemischt, so fällt ein Großteil des Enzalutamides aus, da dessen Sättigungskonzentration überschritten wird. Die resultierende Mischung enthält 50% Eisessig und eine Gesamtmenge von 50 mg/ml Enzalutamide. Davon werden 2,04 mg/ml in der Mutterlauge gelöst vorliegen (Tabelle 3, Nr. 2, Eisessig); dies entspricht 4,09 % der Gesamtmenge von 50 mg/ml (Tabelle 4, Nr. 2, Aceton). Diese gelöste Menge an Enzalutamide (4,09 % der Gesamtmenge) führt zu einem im Vergleich zu Aceton deutlich verlangsamten Wachstum der Partikel.

Beispiel 3

Die thermodynamische Sättigungslöslichkeit von APR in verschiedene Lösungsmitteln und deren Mischungen mit Wasser wurde nach dem vorstehend beschriebene Verfahren bestimmt. Die Ergebnisse sind in Tabelle 5 angegeben.

Tabelle 5: Sättigungslöslichkeit von APR in Tetrahydrofuran, Aceton, Eisessig und in Mischungen mit Wasser

| Nr. | LösungsmittelGehalt der Mischung | Sättigungslöslichkeit von APR | | | | |
|---|---|---|---|---|---|---|
| | | Ameisensäure | Eisessig | THF | Aceton | Ethanol |
| | % | mg/ml | mg/ml | mg/ml | mg/ml | mg/ml |
| 1 | 100 | 718,1 | 395,0 | 296,8 | 37,5 | 22,7 |
| 2 | 50 | 132,3 | 2,226 | 1,286 | 1,848 | 0,920 |
| 3 | 33,3 | 39,28 | 0,382 | 0,400 | 0,317 | 0,080 |
| 4 | 25 | 12,11 | 0,081 | 0,058 | 0,064 | 0,014 |
| 5 | 9,09 | 0,015 | 0,005 | 0,004 | 0,003 | 0,004 |

Mischt man Aceton mit Trifluroessigsäure, so kann die Löslichkeit des Wirkstoffs Aprepitant weiter gesteigert werden auf über 1000 mg/ml.

Tabelle 6: Sättigungslöslichkeit von APR in Mischungen aus Trifluroessigsäure und Aceton

| **Formulierung ID** | **Trifluroessigsäure** | **Aceton** | **Sättigungslöslichkeit von APR** |
|---|---|---|---|
| | Vol-% | Vol-% | mg/ml |
| F27G125 | 20.0 | 80,0 | 873 |
| F23G125 | 33.3 | 66,6 | 1000 |
| F33G125 | 60.0 | 40,0 | 1321 |

Beispiel 4

Herstellung von Nanopartikeln durch Fällung aus Ameisensäure

Aprepitant wurde mit einer Konzentration von 400 mg/mL in Ameisensäure aufgelöst. Als Antisolvens wurde Wasser verwendet, das 140 mg/ml Natriumlaurylsulfat enthielt. Die Mischung von Solvent und Antisolvent erfolgte bei Raumtemperatur in einem MicroJetReaktor mit 400 $\mu$m Düsenöffnung. Der Strom des Solvens betrug 60 ml/min, der des Antisolvens betrug 240 ml/min. Dabei bildeten sich Nanopartikel, die aufgefangen und charakterisiert wurden. Die Partikel hatten eine Größe von 254 nm (z-average) mit Größenverteilung (PDI-Wert) von 0,2.

Die theoretische Endkonzentration des Aprepitants in der Dispersion betrug 60 mg/ml; dieser Wert wurde analytisch mittels HPLC überprüft. Die Suspension enthielt 54,4 mg/ml Aprepitant.

Durchführung eines Tests auf Dispersion

Ziel des Tests ist es, zu überprüfen, ob es möglich ist, 125mg Aprepitant in Form von Nanopartikeln vollständig in 200mL FaSSIF zu (re-)dispergieren; dies würde einer 100%igen Dispersion entsprechen. FaSSIF steht dabei für "Fasted state simulated intestinal fluid". Die Zusammensetzung ist in der folgenden Tabelle 7 angegeben.

Tabelle 7: Zusammensetzung von FaSSIF (mM)

| | FaSSIF |
|---|---|
| pH | 6.5 |
| Taurocholat | 3 |
| Phospholipide | 0.75 |
| Natrium | 148 |
| Chlorid | 106 |
| Phosphat | 29 |
| Essiqsäure | - |

Als Dispersionsmedium wird "FaSSIF" verwendet, das gemäß der Anleitung des Lieferanten (Biorelevant.com Ltd.) hergestellt wird. Nach einer Standzeit von 2 Stunden ist das Medium gemäß Anleitung einsatzfähig.

Basierend auf der theoretischen Konzentration von 60 mg/ml wird das Volumen an Suspension berechnet, das theoretisch 125 mg Aprepitant enthält. Für die oben beschrieben Suspension ID664 sind das 2,083 ml. 200 ml FaSSIF werden in einem 250 ml Becherglas vorgelegt. Das Medium wird mit einer Geschwindigkeit von 700 Umdrehungen pro Minute gerührt. Unter Rühren wird die zuvor berechnete Menge an Nanosuspension ins Becherglas pipettiert. Nach 1 Stunde Rühren wird eine Probe entnommen, durch einen 0,45$\mu$m PTFE-Membranfilter filtriert. 125 mg Aprepitant sind nicht in 200 ml FaSSIF löslich; daher enthält das Filtrat feste Aprepitant-Nanopartikel. Diese werden durch geeignete Verdünnung mit einem organischen Lösungsmittel vollständig gelöst und durch eine geeignete HPLC-Methode quantifiziert. Der gefunden Wert wird relativ zu 0,625 mg/ml (entspricht 125 mg in 200 ml) ausgedrückt. Dies entspricht einer 100%igen Dispersion.

Die Dispersion der Suspension ID664 (hergestellt am 12.11.2018) betrug 83%. Die Dispersion von EMEND beträgt in diesem Test 66%.

Herstellung von Nanopartikeln von Aprepitant durch Fällung aus einer Säure

| | Herstellung einer Suspension unter Verwendung von Eisessig | | | | | | | | Zusammensetzung der Suspension | | | Charakterisierung der Suspension | | |
|---|---|---|---|---|---|---|---|---|---|---|---|---|---|---|
| ID | Solvens | Konz Säure (%) | API (mg/ml) | Antisolvens | SDS (mg/ml) | Solvens Volumen (µl) | AntiSolvens Volumen (µl) | Mischungsverhältnis S/N | Solvens (v/v%) | API (mg/ml) | SDS (mg/ml) | Partikelgröße (nm) | PDI | Dispersion (%) |
| 633 | Eisessig | 100 | 300 | Wasser | 115 | 100 | 400 | 1:4 | 20 | 60 | 92 | 119,8 | 0,144 | 8,2 |
| | Herstellung einer Suspension unter Verwendung von Ameisensäure | | | | | | | | Zusammensetzung der Suspension | | | Charakterisierung der Suspension | | |
| ID | Solvens | Konz Säure (%) | API (mg/ml) | Antisolvens | SDS (mg/ml) | Solvens Volumen (µl) | AntiSolvens Volumen (µl) | Mischungsverhältnis S/N | Solvens (v/v%) | API (mg/ml) | SDS (mg/ml) | Partikelgröße (nm) | PDI | Dispersion (%) |
| 629 | | 98 | 300 | | 36 | 100 | 400 | 1:4 | 20 | 60 | 28,8 | 1290 | 0,358 | |
| 631 | 98 | 98 | 300 | | 115 | 100 | 400 | 1:4 | 20 | 60 | 92 | 230,3 | 0,293 | 30,9 |
| 649 | 98 | 98 | 300 | | 140 | 100 | 400 | 1:4 | 20 | 60 | 112 | 281,2 | 0,164 | 38,7 |
| ExpER_ 660 | | 98 | 60 | | 100 | 100 | 400 | 1:4 | 20 | 12 | 80 | 406,8 | 0,126 | |
| ExpER_ 661 | | 98 | 60 | | 110 | 100 | 400 | 1:4 | 20 | 12 | 88 | 397,5 | 0,246 | |
| ExpER_ 662 | 98 | 98 | 60 | | 120 | 100 | 400 | 1:4 | 20 | 12 | 96 | 203,8 | 0,366 | |
| ExpER_ 663 | | 98 | 60 | | 130 | 100 | 400 | 1:4 | 20 | 12 | 104 | 133,6 | 0,292 | |
| ExpER_ 664 | | 98 | 60 | | 140 | 100 | 400 | 1:4 | 20 | 12 | 112 | 102,1 | 0,226 | |
| ExpER_ 665 | 98 | 98 | 60 | | 150 | 100 | 400 | 1:4 | 20 | 12 | 120 | 113,4 | 0,195 | |
| 660 | | 98 | 300 | | 100 | 100 | 400 | 1:4 | 20 | 60 | 80 | 282,8 | 0,547 | 25,3 |
| 661 | | 98 | 300 | | 110 | 100 | 400 | 1:4 | 20 | 60 | 88 | 327,8 | 0,397 | 27,4 |
| 662 | | 98 | 300 | | 120 | 100 | 400 | 1:4 | 20 | 60 | 96 | 247,8 | 0,225 | 31,5 |
| 663 | | 98 | 300 | | 130 | 100 | 400 | 1:4 | 20 | 60 | 104 | 301 | 0,316 | 60,9 |

(fortgesetzt)

| | Herstellung einer Suspension unter Verwendung von Ameisensäure | | | | | | | | Zusammensetzung der Suspension | | | Charakterisierung der Suspension | | |
|---|---|---|---|---|---|---|---|---|---|---|---|---|---|---|
| ID | Solvens | Konz Säure (%) | API (mg/ml) | Antisolvens | SDS (mg/ml) | Solvens Volumen (µl) | AntiSolvens Volumen (µl) | Mischungsverhältnis S/N | Solvens (v/v%) | API (mg/ml) | SDS (mg/ml) | Partikelgröße (nm) | PDI | Dispersion (%) |
| 664 | | 98 | 300 | | 140 | 100 | 400 | 1:4 | 20 | 60 | 112 | 271,3 | 0,299 | |
| 664_Lyo | | 98 | 300 | | 140 | 100 | 400 | 1:4 | 20 | 60 | 112 | 243,4 | 0,403 | 64,7 |
| 665 | 98 | 98 | 300 | | 150 | 100 | 400 | 1:4 | 20 | 60 | 120 | 249 | 0,269 | 51,8 |
| 671 | 98 | 98 | 300 | | 150 | 100 | 100 | 1:1 | 50 | 150 | 75 | 614,1 | 0,252 | 12,7 |
| 670 | | 98 | 300 | | 150 | 100 | 200 | 1:2 | 33 | 100 | 100 | 280 | 0,527 | 14,9 |
| 669 | | 98 | 300 | | 150 | 100 | 300 | 1:3 | 25 | 75 | 112,5 | 313,5 | 0,239 | 20,8 |
| 665 | | 98 | 300 | | 150 | 100 | 400 | 1:4 | 20 | 60 | 120 | 249 | 0,269 | 51,8 |

## Patentansprüche

1. Verfahren zur Herstellung von Nanopartikeln, enthaltend mindestens einen pharmazeutisch aktiven Wirkstoff, umfassend die Schritte:

   a) Bereitstellen einer Lösung des mindestens einen pharmazeutisch aktiven Wirkstoffs in einer Säure mit einem pks ≤ 5;
   b) Bereitstellen eines Antilösungsmittels für den mindestens einen pharmazeutisch aktiven Wirkstoff; und
   c) Ausfällen von Nanopartikeln, umfassend den mindestens einen pharmazeutisch aktiven Wirkstoff, durch Mischen der Lösung aus Schritt a) mit dem Antilösungsmittel aus Schritt b), um eine Suspension von Nanopartikeln zu erhalten, wobei die pharmazeutisch aktiven Wirkstoffe Tacrolimus und Nilotinib ausgeschlossen sind.

2. Verfahren gemäß Anspruch 1, **dadurch gekennzeichnet, dass** das Mischen in Schritt c) das Kollidieren eines Stroms der Lösung a) mit einem Strom des Antilösungsmittels b) umfasst.

3. Verfahren gemäß irgendeinem der vorhergehenden Ansprüche, **dadurch gekennzeichnet, dass** das Mischen in Schritt c) das Kollidieren eines Stroms der Lösung a) mit einem Strom des Antilösungsmittels b) umfasst, wobei die beiden Ströme bei einer Geschwindigkeit von 1 m/s bis 500 m/s, vorzugsweise von 1 m/s bis 200 m/s, kollidieren.

4. Verfahren gemäß irgendeinem der vorhergehenden Ansprüche, **dadurch gekennzeichnet, dass** die Säure mit einem pks ≤ 5 im Wesentlichen wasserfrei ist.

5. Verfahren gemäß irgendeinem der vorhergehenden Ansprüche, **dadurch gekennzeichnet, dass** die Säure mit einem pks ≤ 5 eine organische Säure, vorzugsweise im Wesentlichen wasserfrei, mit einem pks ≤ 5 ist.

6. Verfahren gemäß irgendeinem der vorhergehenden Ansprüche, **dadurch gekennzeichnet, dass** die Säure mit einem pks ≤ 5 Eisessig und/oder Ameisensäure, vorzugsweise im Wesentlichen wasserfrei, ist.

7. Verfahren gemäß irgendeinem der vorhergehenden Ansprüche, **dadurch gekennzeichnet, dass** das Antilösungsmittel Wasser umfasst oder Wasser ist.

8. Verfahren gemäß irgendeinem der vorhergehenden Ansprüche, **dadurch gekennzeichnet, dass** die Konzentration des mindestens einen pharmazeutisch aktiven Wirkstoffs in der Suspension mindestens 50 mg/ml, vorzugsweise 80 mg/ml bis 300 mg/ml, besonders bevorzugt 50 mg/ml bis 100 mg/ml beträgt

9. Verfahren gemäß irgendeinem der vorhergehenden Ansprüche, **dadurch gekennzeichnet, dass** der mindestens eine pharmazeutisch aktive Wirkstoff Itraconazol, Enzalutamide und/oder Aprepitant, umfasst.

10. Verfahren gemäß irgendeinem der vorhergehenden Ansprüche, **dadurch gekennzeichnet, dass** das Volumenverhältnis der Lösung, umfassend den mindestens einen pharmazeutisch aktiven Wirkstoff aus Schritt a) zu dem Antilösungsmittel aus Schritt b) von 1:1 bis 1:10, vorzugsweise von 1:1 bis 1:5, beträgt.

11. Verfahren gemäß irgendeinem der vorhergehenden Ansprüche, **dadurch gekennzeichnet, dass** das Antilösungsmittel aus Schritt b) mindestens ein ionisches und/oder nicht-ionische Detergenz, vorzugsweise Natruimdodecylsulfat, umfasst.

12. Verfahren gemäß irgendeinem der vorhergehenden Ansprüche, **dadurch gekennzeichnet, dass** die Nanoartikel eine durchschnittliche Partikelgröße, gemessen mittels dynamischer Lichtstreuung, von 10 bis 500 nm, vorzugsweise von 10 bis 200 nm aufweisen.

13. Verfahren gemäß irgendeinem der vorhergehenden Ansprüche, **dadurch gekennzeichnet, dass** die Nanoartikel einen Polydispersitätsindex von ≤ 0,4 , bevorzugt von ≤ 0,2, aufweisen.

14. Nanopartikel, erhältlich durch das Verfahren gemäß irgendeinem der Ansprüche 1 bis 13.

15. Pharmazeutische Zusammensetzung, umfassend die Nanopartikel gemäß Anspruch 14.

Figur 1

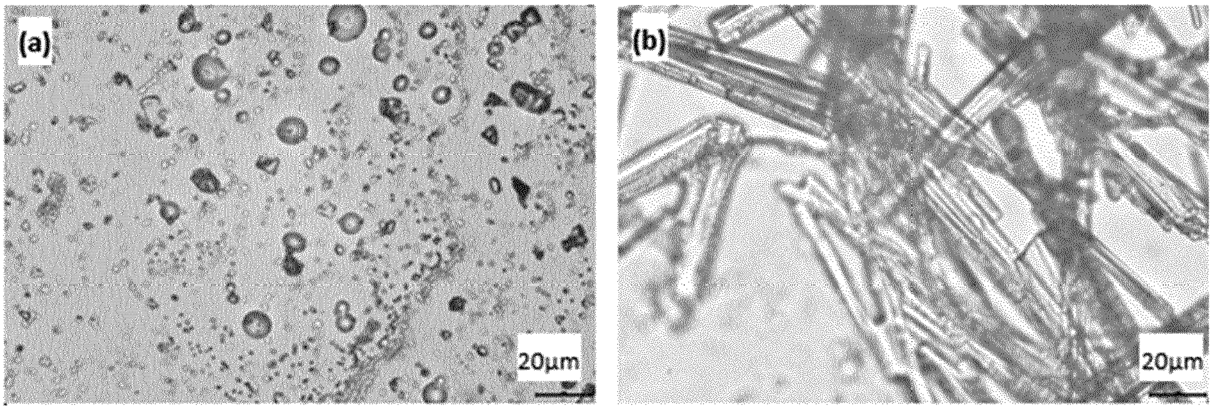

Figur 2

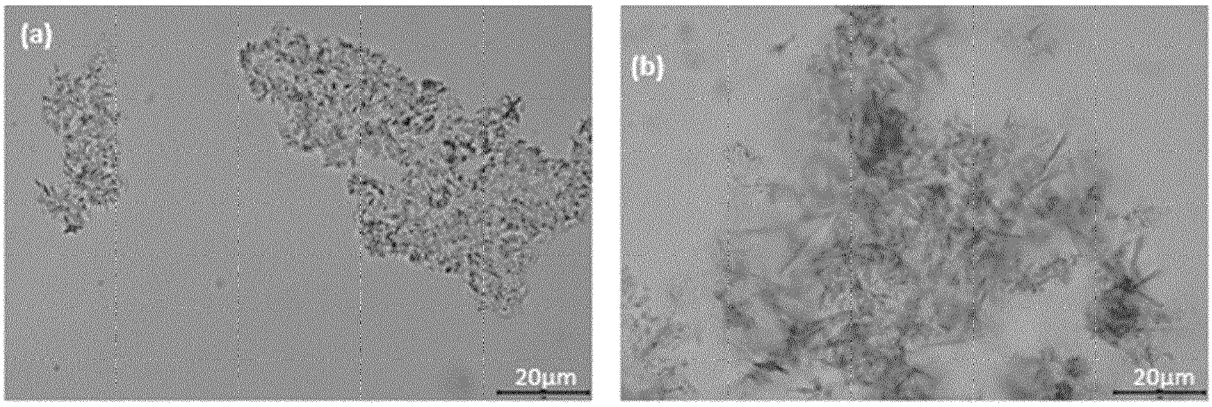

Europäisches Patentamt
European Patent Office
Office européen des brevets

# EUROPÄISCHER RECHERCHENBERICHT

Nummer der Anmeldung
EP 20 17 6307

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X | ZHANG J Y ET AL: "Preparation of amorphous cefuroxime axetil nanoparticles by controlled nanoprecipitation method without surfactants", INTERNATIONAL JOURNAL OF PHARMACEUTICS, ELSEVIER, NL, Bd. 323, Nr. 1-2, 12. Oktober 2006 (2006-10-12), Seiten 153-160, XP027972600, ISSN: 0378-5173 [gefunden am 2006-10-12] * Seite 154, rechte Spalte, Absatz 3; Tabelle 1 * * Seite 155, linke Spalte, letzter Absatz - Seite 155, rechte Spalte, Absatz 2 * ----- | 1,2,5-8, 12-15 | INV. A61K9/14 A61K9/51 |
| X | YING XU ET AL: "Enhanced dissolution and oral bioavailability of aripiprazole nanosuspensions prepared by nanoprecipitation/homogenization based on acid-base neutralization", INTERNATIONAL JOURNAL OF PHARMACEUTICS, Bd. 438, Nr. 1-2, 1. November 2012 (2012-11-01), Seiten 287-295, XP055741848, NL ISSN: 0378-5173, DOI: 10.1016/j.ijpharm.2012.09.020 * Seite 288, rechte Spalte, Absatz 1-2 * ----- | 1,2,7, 11-15 | RECHERCHIERTE SACHGEBIETE (IPC) A61K |

-/--

Der vorliegende Recherchenbericht wurde für alle Patentansprüche erstellt

1

| Recherchenort | Abschlußdatum der Recherche | Prüfer |
|---|---|---|
| Den Haag | 20. Oktober 2020 | Schwald, Claudia |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
&: Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 20 17 6307

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | Ossama Y Abdallah ET AL: "Formulation and characterization of itraconazole oral nano-suspension: methyl cellulose as a promising stabilizer", , 23. März 2015 (2015-03-23), XP055741864, Gefunden im Internet: URL:https://www.researchgate.net/publication/301286317_Formulation_and_characterization_of_itraconazole_oral_nano-suspension_methyl_cellulose_as_a_promising_stabilizer [gefunden am 2020-10-20] * Seite 1, rechte Spalte, letzter Absatz - Seite 2, linke Spalte, Absatz 1 * * Beispiel 1 * ----- | 1,7-9, 11-15 | |
| A | WO 2019/055539 A1 (PRUDHOMME ROBERT K [US]; FENG JIE [US] ET AL.) 21. März 2019 (2019-03-21) * Ansprüche 1, 37; Beispiel 8 * ----- | 1-15 | |
| A | DE 10 2009 008478 A1 (PHAST GES FUER PHARMAZEUTISCHE [DE]) 19. August 2010 (2010-08-19) * Absätze [0006], [0009]; Beispiel 1 * ----- | 1-15 | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| Den Haag | 20. Oktober 2020 | Schwald, Claudia |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Seite 2 von 2

**ANHANG ZUM EUROPÄISCHEN RECHERCHENBERICHT ÜBER DIE EUROPÄISCHE PATENTANMELDUNG NR.** EP 20 17 6307

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-10-2020

| Im Recherchenbericht angeführtes Patentdokument | Datum der Veröffentlichung | Mitglied(er) der Patentfamilie | Datum der Veröffentlichung |
|---|---|---|---|
| WO 2019055539 A1 | 21-03-2019 | US 2020206136 A1 | 02-07-2020 |
| | | WO 2019055539 A1 | 21-03-2019 |
| DE 102009008478 A1 | 19-08-2010 | BR PI1008359 A2 | 23-02-2016 |
| | | CA 2751625 A1 | 19-08-2010 |
| | | CN 102316853 A | 11-01-2012 |
| | | CN 105125502 A | 09-12-2015 |
| | | DE 102009008478 A1 | 19-08-2010 |
| | | EP 2395978 A2 | 21-12-2011 |
| | | JP 5624059 B2 | 12-11-2014 |
| | | JP 2012517403 A | 02-08-2012 |
| | | KR 20110118714 A | 31-10-2011 |
| | | US 2011294770 A1 | 01-12-2011 |
| | | WO 2010091683 A2 | 19-08-2010 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1165224 B1
- EP 1165224 A2
- WO 2018234217 A1
- WO 2003049718 A


### In der Beschreibung aufgeführte Nicht-Patentliteratur


- **DUNCAN, R. et al.** European Science Foundation, Nanomedicine - An ESF - European Medical Research Councils (EMRC) Forward Look report. European Science Foundation: Straßburg, 2004, 1-48
- **COUVREUR, P. ; C. VAUTHIER.** Nanotechnology: intelligent design to treat complex disease. *Pharm Res,* 2006, vol. 23 (7), 1417-50
- **DAMGE, C. ; C.P. REIS ; P. MAINCENT.** Nanoparticle strategies for the oral delivery of insulin. *Expert Opin Drug Deliv,* 2008, vol. 5 (1), 45-68
- **SCHAFER-KORTING, M. ; W. MEHNERT ; H.C. KORTING.** Lipid nanoparticles for improved topical application of drugs for skin diseases. *Adv Drug Deliv Rev,* 2007, vol. 59 (6), 427-43
- **VAUTHIER, C. ; P. COUVREUR.** Developing nanoparticle drug carriers. *Pharmaceutical technology europe,* 2007, vol. 1, 35-42